# EUROPÄISCHE PATENTSCHRIFT

(11) **EP 3 404 437 B1**
(45) Veröffentlichungstag und Bekanntmachung des Hinweises auf die Patenterteilung: **06.11.2024**
(21) Anmeldenummer: 17171531.1
(22) Anmeldetag: 17.05.2017
(51) Int. Cl.: G01R 33/565, G01R 33/561

(54) **BEWEGUNGSKORREKTUR BEI DER MAGNETRESONANZBILDGEBUNG**
MOTION CORRECTION IN MAGNETIC RESONANCE IMAGING
CORRECTION DE MOUVEMENT DANS L'IMAGERIE PAR RÉSONANCE MAGNÉTIQUE

(43) Veröffentlichungstag der Anmeldung: 21.11.2018
(73) Patentinhaber: Siemens Healthineers AG, 91301 Forchheim (DE)
(72) Erfinder: Forman, Christoph, 91052 Erlangen (DE); Nickel, Marcel Dominik, 91074 Herzogenaurach (DE); Speier, Peter, 91056 Erlangen (DE)
(74) Vertreter: Siemens Healthineers Patent Attorneys

(56) Entgegenhaltungen:
- US-A1- 2013 197 347
- US-A1- 2015 015 691
- M. SALMAN ASIF ET AL: "Motion-adaptive spatio-temporal regularization for accelerated dynamic MRI", MAGNETIC RESONANCE IN MEDICINE, vol. 70, no. 3, 6 November 2012 (2012-11-06), pages 800 - 812, XP055159307, ISSN: 0740-3194, DOI: 10.1002/mrm.24524

## Beschreibung

Die Erfindung betrifft ein Verfahren zum Rekonstruieren von dynamischen Bilddaten. Überdies betrifft die Erfindung auch eine Bildrekonstruktionseinrichtung. Weiterhin betrifft die Erfindung ein Magnetresonanzsystem.

Mit Hilfe moderner bildgebender Verfahren werden häufig zwei- oder dreidimensionale Bilddaten oder Zeitserien von Bilddaten erzeugt, die zur Visualisierung eines abgebildeten Untersuchungsobjekts genutzt werden können. Bildgebende Systeme, die auf einem Verfahren der Magnetresonanzmessung, insbesondere von Kernspins basieren, sogenannte Magnetresonanztomographen, haben sich durch vielfältige Anwendungen erfolgreich etabliert und bewährt. Bei dieser Art der Bildakquisition wird meist ein statisches Grundmagnetfeld B₀, das zur Anfangsausrichtung und Homogenisierung von zu untersuchenden magnetischen Dipolen dient, zur Ortsauflösung des bildgebenden Signals mit einem schnell geschalteten Magnetfeld, dem sogenannten Gradientenfeld, überlagert. Zur Bestimmung von Materialeigenschaften eines abzubildenden Untersuchungsobjekts wird die Dephasierung bzw. Relaxationszeit nach einer Auslenkung der Magnetisierung aus der Anfangsausrichtung ermittelt, sodass verschiedene materialtypische Relaxationsmechanismen bzw. Relaxationszeiten identifiziert werden können.

Die Auslenkung erfolgt meist durch eine Anzahl von HF-Pulsen und die Ortsauflösung beruht dabei auf einer zeitlich festgelegten Manipulation der ausgelenkten Magnetisierung mit Hilfe des Gradientenfeldes in einer sogenannten Messsequenz bzw. Ansteuersequenz, welche eine genaue zeitliche Abfolge von HF-Pulsen, der Änderung des Gradientenfeldes (durch Aussenden einer Schaltsequenz von Gradientenpulsen) sowie der Erfassung von Messwerten festlegt. Neben der Relaxation gibt es noch eine Reihe weiterer Mechanismen zur Kontrastbildung, wie zum Beispiel die Flussmessung und die Diffusionsbildgebung.

Typischerweise erfolgt eine Zuordnung zwischen gemessener Magnetisierung - aus der die erwähnten Materialeigenschaften abgeleitet werden können - und einer Ortskoordinate der gemessenen Magnetisierung im Ortsraum, in dem das Untersuchungsobjekt angeordnet ist, mit Hilfe eines Zwischenschritts. In diesem Zwischenschritt werden erfasste Magnetresonanz-Rohdaten an Auslesepunkten im sogenannten "k-Raum" angeordnet, wobei die Koordinaten des k-Raums als Funktion des Gradientenfeldes kodiert sind. Der Betrag der Magnetisierung (insbesondere der Quermagnetisierung, in einer Ebene quer zum vorbeschriebenen Grundmagnetfeld bestimmt) an einem bestimmten Ort des Untersuchungsobjekts kann aus den k-Raum-Daten mit Hilfe einer Fourier-Transformation ermittelt werden. Anders ausgedrückt, werden die k-Raum-Daten (Magnitude und Phase) benötigt, um eine Signalstärke des Signals und gegebenenfalls dessen Phase im Ortsraum zu berechnen.

Die Magnetresonanzbildgebung wird auch bei der Bildaufnahme von bewegten Untersuchungsbereichen verwendet. Beispiele dafür sind die MR-Angiographie und die dynamische kontrastverstärkte Magnetresonanzbildgebung (DCE-MRI, DCE-MRI = dynamic contrast enhanced magnetic resonance imaging = dynamische kontrastverstärkte Magnetresonanzbildgebung), die beispielhaft in den Druckschriften DE 102009036237 A1 oder US 8195275 B2 und beschrieben werden. Bei der Akquisition von Rohdaten werden zu bestimmten Zeitpunkten nur unterabgetastete Rohdaten erfasst, so dass zur Rekonstruktion von Bilddaten zu einem bestimmten Zeitpunkt auch Daten von anderen Zeitpunkten herangezogen werden müssen, die jedoch in einem anderen Bewegungszustand des Untersuchungsbereichs aufgenommen werden. Um Rohdaten, welche zu unterschiedlichen Zeitpunkten akquiriert wurden, trotzdem gemeinsam nutzen zu können, muss herkömmlich angenommen werden, dass Veränderungen in dem abzubildenden Bereich zwischen den einzelnen Zeitpunkten sich nur unwesentlich auf die Abbildung auswirken. Bei einer stärkeren Dynamik im Bildbereich ist diese Annahme jedoch nicht mehr gegeben, so dass sich in den rekonstruierten Bildern Bewegungsartefakte zeigen.

In der Literatur werden Ansätze beschrieben, die Bewegung in der Bildrekonstruktion mit zu berücksichtigen. Dabei kann zum Beispiel durch eine Anwendung von Deformationsfeldern eine höhere Konsistenz der zu verschiedenen Zeitpunkten akquirierten Rohdaten und der daraus rekonstruierten Bilddaten erreicht werden. Einige Ansätze berücksichtigen die Bewegung bei einer iterativen Rekonstruktion von Bilddaten. Dabei wird jedoch nur eine Dimension, d.h. entweder nur die Zeit oder nur die unterschiedlichen Bewegungszustände mitberücksichtigt. Beispielsweise werden bei einer zeitaufgelösten Bildgebung die Bewegungsfelder bei einer ersten Rekonstruktion von Bilddaten mitberechnet und bei einer nachfolgenden zweiten Rekonstruktion mitberücksichtigt.

In M. Salman Asif et al: "Motion-adaptive spatio-temporal regularization for accelerated dynamic MRI", MAGNETIC RESONANCE IN MEDICINE, Bd. 70, Nr. 3, 6. November 2012 (2012-11-06), Seiten 800-812, XP055159307, ISSN: 0740-3194, DOI: 10.1002/mrm.24524) wird eine Rekonstruktion im Rahmen einer MR-Bildgebung beschrieben, wobei räumlich konstante Deformationsfelder zum Einsatz kommen.

In DE 10 2013 205 832 A1 wird ein MR-Bildgebungsverfahren mit einer Bildrekonstruktion beschrieben, wobei Deformationsfelder in Abhängigkeit von der Atembewegung eines Patienten zur Bildkorrektur ermittelt werden.

Es ist somit eine Aufgabe der vorliegenden Erfindung, ein Verfahren zur Rekonstruktion von dynamischen MR-Bilddaten zu entwickeln, mit dem eine verbesserte Korrektur von Bewegungsartefakten möglich ist.

Diese Aufgabe wird durch ein Verfahren zum Rekonstruieren von dynamischen Bilddaten gemäß Patentanspruch 1, eine Bildrekonstruktionseinrichtung gemäß Patentanspruch 8 und durch ein Magnetresonanzbildgebungssystem gemäß Patentanspruch 9 gelöst.

Bei dem erfindungsgemäßen Verfahren zum Rekonstruieren von dynamischen Bilddaten werden zeitabhängig Rohdaten von einem Untersuchungsbereich akquiriert, wobei zumindest ein Teil der Rohdaten unterschiedlichen Werten von Bewegungsparametern zugeordnet sind. Als dynamische Bilddaten sollen in diesem Zusammenhang Bilddaten von einem bewegten Objekt verstanden werden, die über einen vorbestimmten Zeitraum hinweg aufgenommen werden. Unter dem Begriff "Bewegungsparameter" sollen in diesem Zusammenhang Größen verstanden werden, welche einen Einfluss auf einen Bewegungszustand haben. Beispiele hierfür sind zum Beispiel die Zeit, der Atemzustand oder der Herzbewegungszustand. "Zeitabhängiges Akquirieren" soll sich darauf beziehen, dass einzelnen k-Raumpunkten der Rohdaten unterschiedliche Zeitpunkte zugeordnet werden und auf die berechneten Zeitpunkte eine Menge von hintereinander gemessenen k-Raumpunkten zusammengefasst wird.

Um Informationen über eine Zuordnung von Rohdaten zu einzelnen Bewegungszuständen zu gewinnen, können zum Beispiel zusätzlich Navigator-Rohdaten akquiriert und zu Navigator-Bilddaten rekonstruiert werden. Die Navigator-Bilddaten kennzeichnen Bewegungszustände des abzubildenden Untersuchungsbereichs. Auf Basis der nun bekannten Bewegungszustände und der akquirierten Rohdaten können dann Rohdaten einzelnen Bewegungszuständen zugeordnet werden, d.h., es werden mithin nach Bewegungszustand geordnete Rohdaten erzeugt.

Auf Basis der akquirierten Rohdaten werden erste zeitabhängige Bilddaten rekonstruiert. D.h. es werden Bilddaten für mehrere unterschiedliche Zeitpunkte rekonstruiert. Auf Basis der ersten Bilddaten werden darauf in Abhängigkeit von mindestens zwei zeitabhängigen Bewegungsparametern Deformationsfelder ermittelt. Deformationsfelder geben Auskunft über eine Verschiebung eines Punkts eines Untersuchungsbereichs in Abhängigkeit von den genannten Bewegungsparametern, beispielsweise der Zeit und dem Atemzustand oder dem Herzbewegungszustand. Schließlich werden korrigierte Bilddaten auf Basis der Deformationsfelder der Rohdaten und der ersten Bilddaten ermittelt.

Mit Hilfe der Deformationsfelder werden Teile der ersten Bilddaten, welche unterschiedlichen Bewegungsparameterwerten zugeordnet sind, aufeinander registriert, um für jeden Parameterwert mehr Bildinformationen zu erhalten. Auf Basis der genaueren Bildinformation lassen sich dann, beispielsweise im Rahmen einer Iteration, beispielsweise einer iterativen Bildrekonstruktion, verbesserte Deformationsfelder erzeugen, mit deren Hilfe wiederum exaktere Bilddaten, d.h. "korrigierte" Bilddaten rekonstruiert werden können. Mithin können auch bei starker Unterabtastung, wie sie bei Magnetresonanz-Bildaufnahmen von bewegten Objekten üblicherweise vorkommt, Bilddaten mit verbesserter Genauigkeit bzw. mit reduzierten Artefakten erzeugt werden.

Die erfindungsgemäße Bildrekonstruktionseinrichtung umfasst eine Eingangsschnittstelle zum Empfangen von zeitabhängig akquirierten Rohdaten von einem Untersuchungsbereich, wobei zumindest ein Teil der Rohdaten unterschiedlichen Werten von Bewegungsparametern zugeordnet sind. Teil der Bildrekonstruktionseinrichtung ist auch eine Rekonstruktionseinheit zum Rekonstruieren von ersten zeitabhängigen Bilddaten auf Basis der akquirierten Rohdaten. Die erfindungsgemäße Bildrekonstruktionseinrichtung umfasst außerdem eine Deformationsfeld-Ermittlungseinheit zum Ermitteln von Deformationsfeldern auf Basis der ersten Bilddaten in Abhängigkeit von mindestens zwei zeitabhängigen Bewegungsparametern und eine Korrektureinheit zum Erzeugen von korrigierten Bilddaten auf Basis der Deformationsfelder, der Rohdaten und der ersten Bilddaten.

Das erfindungsgemäße Magnetresonanzbildgebungssystem umfasst eine Steuereinrichtung, welche zur Steuerung des Magnetresonanzbildgebungssystems unter Nutzung des erfindungsgemäßen Verfahrens ausgebildet ist. Dazu weist das erfindungsgemäße Magnetresonanzbildgebungssystem die erfindungsgemäße Bildrekonstruktionseinrichtung auf.

Die wesentlichen Komponenten der erfindungsgemäßen Bildrekonstruktionseinrichtung können zum überwiegenden Teil in Form von Softwarekomponenten ausgebildet sein. Dies betrifft insbesondere die Rekonstruktionseinheit, die Deformationsfeld-Ermittlungseinheit und auch die Korrektureinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Insbesondere kann die erfindungsgemäße Bildrekonstruktionseinrichtung Teil eines Benutzerterminals bzw. von einer Steuereinrichtung eines Magnetresonanzbildgebungssystems sein. Eine weitgehend softwaremäßige Realisierung hat den Vorteil, dass auch schon bisher verwendete Bildrekonstruktionseinrichtungen bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines Magnetresonanzbildgebungssystems ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zur Steuereinrichtung und/oder zur Speicherung an oder in der Steuereinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen. Die Rechnereinheit kann zum Beispiel Teil eines Terminals oder der Steuereinrichtung des Magnetresonanzbildgebungssystems sein. Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden, sofern der dadurch erhaltene Gegenstand in den durch die Ansprüche definierten Schutzumfang der Erfindung fällt.

In einer Ausgestaltung des erfindungsgemäßen Verfahrens erfolgt das Erzeugen der korrigierten Bilddaten auf Basis einer iterativen Rekonstruktion, bei der die Deformationsfelder zur Erzeugung von korrigierten Rohdaten genutzt werden. Auf Basis der Deformationsfelder werden zusätzliche bisher nicht vorhandene Rohdaten erzeugt und/oder bereits vorhandene Rohdaten anderen Werten der Bewegungsparameter zugeordnet. Die zusätzlich erzeugten Rohdaten, welche für einen oder mehrere Sätze von Werten von Bewegungsparametern Rohdaten zur Verfügung stehen, können dazu genutzt werden Rohdaten, welche Bewegungszuständen falsch zugewiesen wurden, den korrekten Bewegungszuständen zuzuordnen. Auf diese Weise kann die Bildqualität verbessert bzw. es können Bewegungsartefakte reduziert werden. Deformationsfelder werden auch über eine Optimierung bestimmt. Als Startpunkt kann man ein bekanntes Deformationsfeld setzen. Man setzt in der Regel einfach die Anzahl der Iterationen in der Optimierung der Deformationsfelder fest. Das "Ermitteln" von Deformationsfeldern soll also auch das Optimieren der Deformationsfelder umfassen. Deformationsfelder und Bilddaten können auch gleichzeitig bzw. in einem gemeinsamen Optimierungsvorgang optimiert werden.

In einer Ausgestaltung des erfindungsgemäßen Verfahrens umfasst die Akquisition der Rohdaten eine kartesische, potenziell unvollständige Abtastung. Als unvollständige Abtastung soll in diesem Zusammenhang eine Unterabtastung verstanden werden. Eine solche Abtastung kann zum Beispiel bei einer Leberabbildung besonders vorteilhaft eingesetzt werden, da bei einer solchen Bildgebung eine Ausrichtung der Bilddarstellungen in Kopf-Fußrichtung bzw. in Anterior-Posterior-Richtung erfolgt.

In einer bevorzugten Variante des erfindungsgemäßen Verfahrens umfasst das Ermitteln von Deformationsfeldern eine Registrierung von Bilddaten mit zumindest teilweise unterschiedlichen Werten der zeitabhängigen Bewegungsparameter. Mit Hilfe der Registrierungen werden Verschiebungen in dem Untersuchungsbereich ermittelt. Auf diese Weise lassen sich Bildinformationen, welche bestimmten Werten von Bewegungsparametern zugeordnet sind, auch anderen Werten zuordnen, so dass die Bildqualität für einzelne Wertetupel von Bewegungsparametern verbessert wird.

Erfindungsgemäß handelt es sich bei den mindestens zwei unterschiedlichen zeitabhängigen Bewegungsparametern um die Zeit und einen Atemzustand oder um die Zeit und einen Herzbewegungszustand.

Vorteilhaft werden bei dem erfindungsgemäßen Verfahren mehrere Bewegungsparameter berücksichtigt. Dabei können zum Beispiel mit Hilfe der Deformationsfelder Rohdaten oder Bilddaten, welche demselben Bewegungszustand, aber einem unterschiedlichen Zeitpunkt zugeordnet sind, kombiniert werden und so die Bildqualität verbessert werden.

Erfindungsgemäß werden für das Erzeugen von korrigierten Bilddaten auf Basis der Deformationsfelder für unterschiedliche Werte der mindestens zwei zeitabhängigen Bewegungsparameter synthetisierte Rohdaten ermittelt. Mit Hilfe der Deformationsfelder können Bilddaten korrigierten Positionen zugeordnet werden. Auf Basis der korrigierten Zuordnung lassen sich dann synthetisierte Rohdaten erzeugen, mit deren Hilfe wiederum korrigierte Bilddaten rekonstruiert werden können.

Erfindungsgemäß werden auf Basis der synthetisierten Rohdaten die akquirierten Rohdaten geänderten Werten von Bewegungsparametern neu zugewiesen. Beispielsweise kann anhand eines Vergleichs der synthetisierten Rohdaten mit den akquirierten Rohdaten eine Falschzuweisung der akquirierten Rohdaten erkannt werden und die akquirierten Rohdaten können dann den korrekten Bewegungszuständen zugewiesen werden. Auf diese Weise können auf Deformation beruhende Effekte, welche zu fehlerhaften Zuweisungen der Rohdaten zu einzelnen Bewegungszuständen führen, kompensiert werden. Fehlerhafte Zuweisungen können auch durch eine ungenaue Messung von Bewegungszuständen, beispielsweise eine eindimensionale Navigation, zustande kommen. Auch diese Fehler können durch eine nachträgliche Korrektur bei der Zuweisung der Rohdaten zu einzelnen Bewegungszuständen korrigiert werden.

Vorzugsweise erfolgt dabei die Zuweisung zu geänderten Werten von Bewegungsparametern in Abhängigkeit davon, ob die den Werten zugeordneten Zustände ähnlich weit voneinander entfernt sind. Anders ausgedrückt, vergleicht man für jeden Messpunkt die ermittelten Rohdaten mittels einer Norm, Abstandsmaß oder Korrelation zu den unterschiedlichen synthetischen Messpunkten/Rohdaten und weist Rohdaten dem Bewegungszustand zu, der näher ist bzw. eine größere Korrelation hat. Man ermittelt also, welche synthetisierten Rohdatenpunkte in einem Abstandsmaß oder mittels Korrelation am nächsten an den gemessenen Rohdatenpunkten liegen.

In einer Variante des erfindungsgemäßen Verfahrens erfolgt die Neuzuweisung der Rohdaten zunächst auf Basis einer Untermenge der akquirierten Rohdaten, vorzugweise in einer vollständig abgetasteten Richtung, beispielsweise in Ausleserichtung, und anschließend erfolgt eine Rekonstruktion der korrigierten Bilddaten auf Basis der gesamten Rohdaten.

In der zweiten Rekonstruktion erfolgt dann die auf Basis der Untermenge ermittelte Zuweisung zwischen Rohdaten und Bewegungszuständen. Hierbei werden dann keine Zustandszuweisungen mehr geändert. Vorteilhaft kann mit Hilfe dieser Vorgehensweise Zeit und Datenkapazität gespart werden. Beispielsweise kann eine erste Rekonstruktion von Bilddaten zunächst nur auf Basis von in Ausleserichtung akquirierten Rohdaten erfolgen. Diese Rohdaten werden dann zur Ermittlung von Deformationsfeldern genutzt, welche wiederum zur Korrektur der Zuweisung der akquirierten Rohdaten verwendet werden.

In einer Ausgestaltung des erfindungsgemäßen Verfahrens werden Rohdaten, welche sich in mindestens einem der mindesten zwei zeitabhängigen Bewegungsparameter unterscheiden, verworfen und die korrigierten Bilddaten werden nur in Abhängigkeit einer Untermenge der mindestens zwei zeitabhängigen Bewegungsparameter erzeugt. Vorteilhaft kann die Rekonstruktionszeit aufgrund des reduzierten Datenverarbeitungsaufwands verkürzt werden.

In einer vollständig abgetasteten Richtung können orthogonale Scheiben bzw. scheibenförmigen Schichten unabhängig voneinanpder rekonstruiert werden. Mithin kann man zur Reduktion des Rechenaufwands die Anzahl der Scheiben bzw. Schichten reduzieren, so dass das rekonstruierte Bildvolumen verringert ist.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zum Rekonstruieren von dynamischen Bilddaten veranschaulicht, das nicht alle Merkmale des in Anspruch 1 definierten Verfahrens enthält,
FIG 2 ein Flussdiagramm, welches ein Verfahren zum Rekonstruieren von dynamischen Bilddaten gemäß einem Ausführungsbeispiel der Erfindung veranschaulicht,
FIG 3 Blockdiagramm, welches eine Bildrekonstruktionseinrichtung gemäß einem Ausführungsbeispiel der Erfindung veranschaulicht,
FIG 4 eine schematische Darstellung eines Magnetresonanzbildgebungssystems gemäß einem Ausführungsbeispiel der Erfindung.

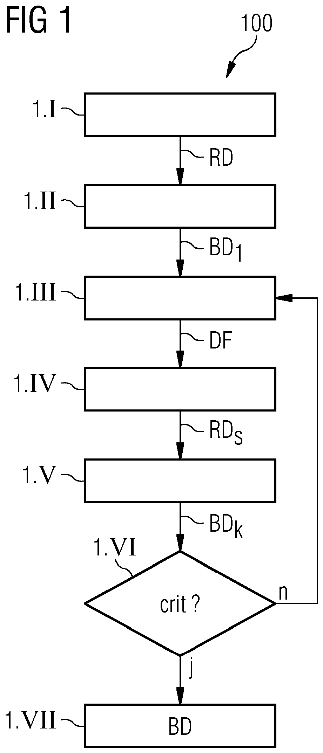

In FIG 1 ist ein Flussdiagramm 100 gezeigt, welches ein Verfahren zum Rekonstruieren von dynamischen Bilddaten veranschaulicht, das nicht alle Merkmale des in Anspruch 1 definierten Verfahrens enthält.

Bei dem Schritt 1.I werden zunächst Rohdaten RD von einem Untersuchungsbereich erfasst. Der Untersuchungsbereich verhält sich während der Akquisition der Rohdaten RD dynamisch, d.h., mindestens ein Teil der Rohdaten RD ist unterschiedlichen Werten von Bewegungsparametern BPₜ zugeordnet. Bei dem Schritt 1.II werden auf Basis der erfassten Rohdaten RD erste zeitabhängige Bilddaten BD1 rekonstruiert.

Bei dem Schritt 1.III erfolgt eine Ermittlung von Deformationsfeldern DF auf Basis der ersten Bilddaten BD1. Die Deformationsfelder DF hängen mindestens von zwei zeitabhängigen Bewegungsparametern, beispielsweise der Zeit t und dem Atembewegungszustand AZ, ab.

Bei dem Schritt 1.IV erfolgt eine Erzeugung von synthetischen Rohdaten RD_{S} anhand der ersten Bilddaten und der Deformationsfelder DF.

Weiterhin werden bei dem Schritt 1.V korrigierte Bilddaten BDₖ auf Basis der synthetischen Rohdaten RDₛ rekonstruiert. Bei dem Schritt 1.VI wird ermittelt, ob die korrigierten Bilddaten BDₖ einem Qualitätskriterium crit entsprechen. Beispielsweise werden die korrigierten Bilddaten BDₖ auf Artefakte geprüft. Für den Fall, dass das Qualitätskriterium noch nicht erreicht ist, was in FIG 1 mit "n" gekennzeichnet ist, wird zu dem Schritt 1.III zurückgekehrt, bei dem erneut Deformationsfelder DF, nun jedoch auf Basis der korrigierten Bilddaten BDₖ, erzeugt werden. Anschließend wird das Verfahren mit den Schritten 1.IV bis 1.VI fortgeführt. Für den Fall dass bei dem Schritt 1.VI ermittelt wurde, dass das Qualitätskriterium crit für die korrigierten Bilder BDₖ erfüllt wurde, was in FIG 1 mit "j" gekennzeichnet ist, so wird zu dem Schritt 1.VII übergegangen, bei dem die zuletzt erzeugten Bilddaten BDₖ als endgültige Bilddaten BD übernommen werden.

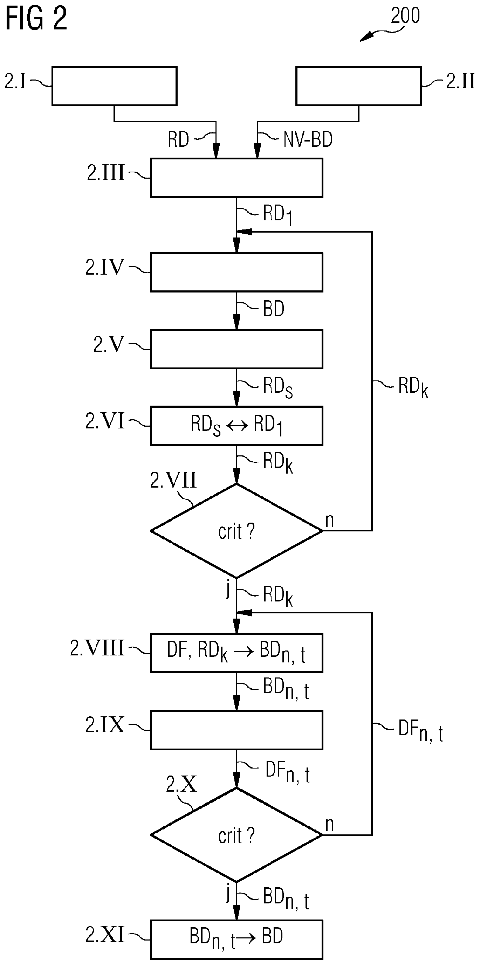

In FIG 2 ist ein Flussdiagramm 200 gezeigt, welches ein Verfahren zum Rekonstruieren von dynamischen Bilddaten gemäß einem Ausführungsbeispiel der Erfindung veranschaulicht.

Bei dem Schritt 2.I werden zunächst Magnetresonanzrohdaten RD von einem Untersuchungsbereich FoV eines Patienten akquiriert. Zusätzlich werden bei dem Schritt 2.II Navigator-Rohdaten NV-RD akquiriert und zu Navigator-Bilddaten NV-BD rekonstruiert. Die Navigator-Bilddaten NV-BD kennzeichnen einen Bewegungszustand des abzubildenden Untersuchungsbereichs FoV. Auf Basis der nun bekannten Bewegungszustände und der Rohdaten RD werden nun bei dem Schritt 2.III Rohdaten RD einzelnen Bewegungszuständen zugeordnet, es werden mithin nach Bewegungszustand geordnete Rohdaten RD₁ erzeugt.

Bei dem Schritt 2.IV werden Bilddaten BD auf Basis der nach Bewegungszuständen geordneten Rohdaten RD₁ rekonstruiert. Dabei werden Rohdaten, welche unterschiedlichen Zeitpunkten, aber denselben Bewegungszuständen zugeordnet sind, miteinander kombiniert. Weiterhin wird bei dem Schritt 2.V für jeden Bewegungszustand n und jeden Zeitpunkt t ein vollständiger Satz von Rohdaten mit zusätzlichen synthetisierten RDₛ im k-Raum erzeugt, welche die aufgrund einer Unterabtastung bei der Rohdatenakquisition fehlenden Rohdaten ersetzen. Bei dem Schritt 2.VI erfolgt dann eine Neuzuordnung der akquirierten Rohdaten RD zu den einzelnen Bewegungszuständen durch Abgleich der Zuordnung der akquirierten Rohdaten RD bzw. der geordneten Rohdaten RD₁ mit der Zuordnung der synthetisierten Rohdaten RDₛ. Dabei werden gegebenenfalls korrigierte bzw. umgeordnete Rohdaten RDₖ erzeugt. Bei dem Schritt 2.VII wird geprüft, ob ein Abbruchkriterium crit erfüllt ist. Für den Fall, dass das Abbruchkriterium noch nicht erfüllt ist, was in FIG 2 mit "n" gekennzeichnet ist, wird zu dem Schritt 2.IV zurückgekehrt und es erfolgt eine erneute Rekonstruktion von Bilddaten BD auf Basis der bei dem Schritt 2.VI korrigierten Rohdaten RDₖ. Nachfolgend werden wieder die Schritte 2.V und 2.VI ausgeführt. Für den Fall, dass bei dem Schritt 2.VII ein Abbruchkriterium crit erfüllt ist, beispielsweise hat ein Zählindex eine Maximalzahl der durchzuführenden Iterationen erreicht, was in FIG 2 mit "j" gekennzeichnet ist, so wird zu dem Schritt 2.VIII übergegangen.

Bei dem Schritt 2.VIII werden Bilddaten BD_{n,t} auf Basis der bei dem Schritt 2.VI ermittelten korrigierten Rohdaten RDₖ, welche nach Bewegungszustand n und Zeitpunkt t geordnet sind, rekonstruiert, wobei die Bilder unterschiedlicher Bewegungszustände unter Anwendung von Deformationsfeldern DF erzeugt werden. Anders ausgedrückt werden die Deformationsfelder DF dazu genutzt, um Bilddaten von unterschiedlichen Bewegungszuständen aufeinander zu registrieren, um so auch bei einer Unterabtastung einzelner Bewegungszustände für jeden der Bewegungszustände eine ausreichende Datenbasis zu erhalten. Als initiales Deformationsfeld DF₀ kann zum Beispiel eine die Identität repräsentierende Matrix genutzt werden. Nachfolgend werden bei dem Schritt 2.IX Deformationsfelder DF_{n,t} zwischen unterschiedlichen Bewegungszuständen und unterschiedlichen Zeiten auf Basis der rekonstruierten Bilddaten BD_{n,t} ermittelt. Für den Fall, dass ein Abbruchkriterium crit nicht erfüllt ist, was in FIG 2 mit "n" gekennzeichnet ist, wird bei dem Schritt 2.X zu dem Schritt 2.VIII zurückgekehrt und es werden wiederum nach Bewegungszustand und Zeit geordnete Bilddaten BD_{n,t} rekonstruiert, diesmal jedoch unter Anwendung der neu erzeugten Deformationsfelder DF_{n,t} zwischen unterschiedlichen Bewegungszuständen n und unterschiedlichen Zeiten t. Für den Fall, dass bei dem Schritt 2.X ein Abbruchkriterium crit für die Iteration erfüllt ist, was in FIG 2 mit "j" kennzeichnet ist, so wird zu dem Schritt 2.XI übergegangen, bei dem die zuletzt rekonstruierten Bilddaten BD_{n,t} als endgültige Bilddaten BD ausgegeben werden.

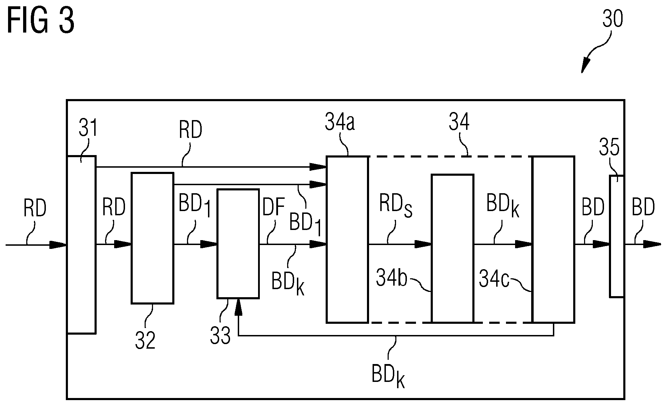

In FIG 3 ist eine Bildrekonstruktionseinrichtung 30 gemäß einem Ausführungsbeispiel der Erfindung veranschaulicht. Die Bildrekonstruktionseinrichtung 30 umfasst eine Eingangsschnittstelle 31, welche dazu eingerichtet ist, zeitabhängig akquirierte Rohdaten RD zu empfangen. Zumindest ein Teil der Rohdaten RD ist unterschiedlichen Werten von Bewegungsparametern BPₜ zugeordnet. Die Rohdaten RD werden an eine Rekonstruktionseinheit 32 übermittelt, welche erste zeitabhängige Bilddaten BD₁ auf Basis der akquirierten Rohdaten RD rekonstruiert. Die Bilddaten BD₁ werden an eine Deformationsfeld-Ermittlungseinheit 33 weitergegeben, welche Deformationsfelder DF auf Basis der ersten Bilddaten BD₁ in Abhängigkeit von mindestens zwei zeitabhängigen Bewegungsparametern BPₜ ermittelt. Die ermittelten Deformationsfelder DF sowie die empfangenen Rohdaten RD und die rekonstruierten Bilddaten BD₁ werden an eine Korrektureinheit 34 übermittelt, welche auf Basis der Deformationsfelder DF, der Rohdaten und der ersten Bilddaten BD1 korrigierte Bilddaten BDₖ ermittelt.

Die Korrektureinheit 34 umfasst im Einzelnen eine Synthetisierungseinheit 34a, welche auf Basis der Deformationsfelder DF, der Rohdaten RD und der Bilddaten BD1 synthetisierte Rohdaten RDₛ erzeugt. Teil der Korrektureinheit 34 ist auch eine Bilddatenrekonstruktionseinheit 34b, welche auf Basis der synthetisierten Rohdaten RDₛ korrigierte Bilddaten BDₖ rekonstruiert. Die korrigierten Bilddaten BDₖ werden an eine Prüfeinheit 34c übermittelt, welche dazu eingerichtet ist, die korrigierten Bilddaten BDₖ bezüglich des Auftretens von Artefakten zu prüfen. Erfüllen die geprüften Bilddaten BDₖ ein vorbestimmtes Qualitätskriterium noch nicht, so werden die korrigierten Bilddaten BDₖ an die Deformationsfeld-Ermittlungseinheit 33 zurückübermittelt, welche auf Basis der korrigierten Bilddaten BDₖ korrigierte Deformationsfelder DF erzeugt, die dann wiederum gemeinsam mit den korrigierten Bilddaten BDₖ an die Korrektureinheit 34 übermittelt werden, von der erneut korrigierte Bilddaten BDₖ erzeugt werden. Für den Fall, dass diese dem genannten Qualitätskriterium entsprechen, werden die zuletzt erzeugten korrigierten Bilddaten BDₖ als die endgültigen Bilddaten BD festgelegt und über eine Ausgabeschnittstelle 35 ausgegeben.

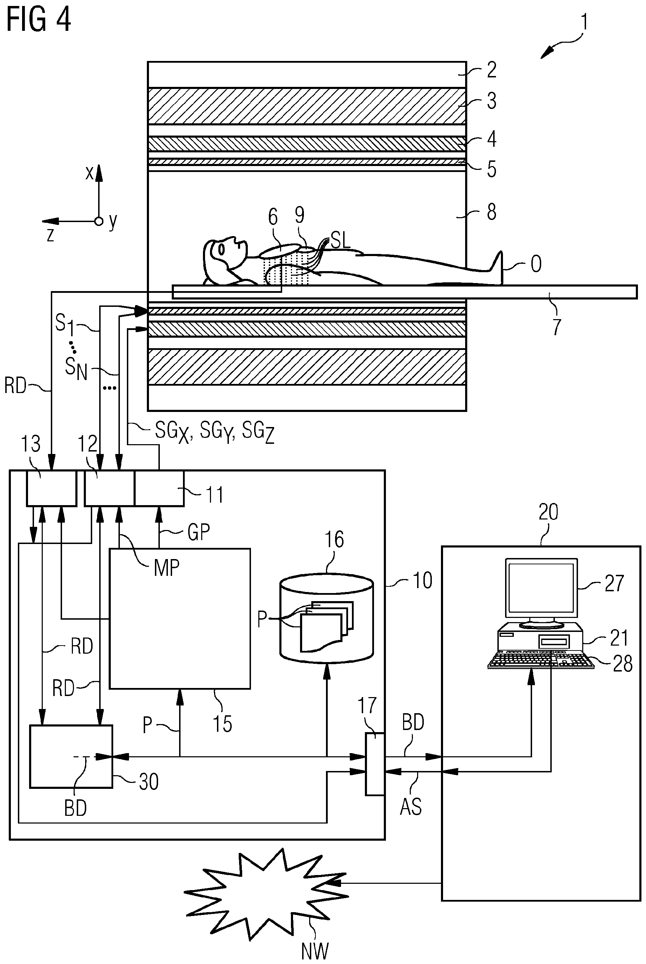

FIG 4 zeigt grob schematisch eine erfindungsgemäße Magnetresonanzanlage bzw. ein Magnetresonanzbildgebungssystem 1. Diese umfasst den eigentlichen Magnetresonanzscanner 2 mit einem darin befindlichen Messraum 8 bzw. Patiententunnel. Eine Liege 7 ist in diesen Patiententunnel 8 hineinfahrbar, so dass ein darauf liegendes Untersuchungsobjekt O (Patient/Proband) während einer Untersuchung an einer bestimmten Position innerhalb des Magnetresonanzscanners 2 relativ zu dem darin angeordneten Magnetsystem und Hochfrequenzsystem gelagert werden kann bzw. auch während einer Messung zwischen verschiedenen Positionen verfahrbar ist.

Wesentliche Komponenten des Magnetresonanzscanners 2 sind ein Grundfeldmagnet 3, ein Gradientensystem 4 mit Gradientenspulen, um beliebige Magnetfeldgradienten in x-, y- und z- Richtung anzulegen, sowie eine Ganzkörper-Hochfrequenzspule 5. Der Empfang von im Untersuchungsobjekt O induzierten Magnetresonanzsignalen kann über die Ganzkörperspule 5 erfolgen, mit der in der Regel auch die Hochfrequenzsignale zur Induzierung der Magnetresonanzsignale ausgesendet werden. Üblicherweise werden diese Signale aber mit beispielsweise auf oder unter das Untersuchungsobjekt O gelegten Lokalspulen 6 empfangen. Alle diese Komponenten sind dem Fachmann grundsätzlich bekannt und daher in FIG 4 nur grob schematisch dargestellt.

Die Ganzkörper-Hochfrequenzspule 5 kann z. B. in Form einer so genannten Birdcage-Antenne eine Anzahl N von einzelnen Antennenstäben aufweisen, die als einzelne Sendekanäle S1, ..., SN separat von einer Steuereinrichtung 10 ansteuerbar sind, d. h. es handelt sich bei dem Magnetresonanztomographiesystem 1 um ein pTX-fähiges System. Es wird aber ausdrücklich darauf hingewiesen, dass das erfindungsgemäße Verfahren auch an klassischen Magnetresonanztomographiegeräten mit nur einem Sendekanal anwendbar ist.

Bei der Steuereinrichtung 10 kann es sich um einen Steuerrechner handeln, der auch aus einer Vielzahl von - gegebenenfalls auch räumlich getrennten und über geeignete Bussysteme bzw. Kabel oder dergleichen untereinander verbundenen - Einzelrechnern bestehen kann. Über eine Terminalschnittstelle 17 ist diese Steuereinrichtung 10 mit einem Terminal 20 verbunden, über das ein Bediener die gesamte Anlage 1 ansteuern kann. Im vorliegenden Fall weist dieses Terminal 20 einen Rechner 21 mit Tastatur 28, einem oder mehreren Bildschirmen 27 sowie weiteren Eingabegeräten wie beispielsweise Maus oder dergleichen auf, so dass dem Bediener eine grafische Benutzeroberfläche zur Verfügung steht.

Die Steuereinrichtung 10 weist u. a. eine Gradienten-Steuereinheit 11 auf, die wiederum aus mehreren Teilkomponenten bestehen kann. Über diese Gradienten-Steuereinheit 11 werden die einzelnen Gradientenspulen mit Steuersignalen SGx, SGy, SGz beschaltet. Hierbei handelt es sich um Gradientenpulse, die während einer Messung an genau vorgesehenen zeitlichen Positionen und mit einem genau vorgegebenen zeitlichen Verlauf gesetzt werden, um das Untersuchungsobjekt O und den zugeordneten k-Raum vorzugsweise in einzelnen Schichten SL gemäß einer Ansteuersequenz AS abzutasten.

Die Steuereinrichtung 10 weist außerdem eine Hochfrequenz-Sende-/Empfangseinheit 12 auf. Diese HF-Sende-/Empfangseinheit 12 besteht ebenfalls aus mehreren Teilkomponenten, um jeweils separat und parallel auf die einzelnen Sendekanäle S₁, ... S_{N}, d. h. in diesem Fall auf die einzeln ansteuerbaren Antennenstäbe der Bodycoil 5, Hochfrequenzpulse aufzugeben. Über die Sende-/Empfangseinheit 12 können auch Magnetresonanzsignale empfangen werden. In diesem Ausführungsbeispiel geschieht dies aber mit Hilfe der Lokalspulen 6. Die mit diesen Lokalspulen 6 empfangenen Rohdaten RD werden von einer HF-Empfangseinheit 13 ausgelesen und verarbeitet. Die hiervon oder von der Ganzkörperspule 5 mittels der HF-Sende-/Empfangseinheit 12 empfangenen Magnetresonanzsignale werden als Rohdaten RD an eine erfindungsgemäße Bildrekonstruktionseinrichtung 30 übergeben, die daraus auf die im Zusammenhang mit FIG 3 beschriebene Art und Weise die Bilddaten BD rekonstruiert und diese in einem Speicher 16 hinterlegt und/oder über die Schnittstelle 17 an das Terminal 20 übergibt, so dass der Bediener sie betrachten kann. Die Bilddaten BD können auch über ein Netzwerk NW an anderen Stellen gespeichert und/oder angezeigt und ausgewertet werden. Sofern die Lokalspulen 6 eine geeignete Umschalteinheit aufweisen, können auch diese an eine HF-Sende-/Empfangseinheit 12 angeschlossen sein, um die Lokalspulen auch zum Senden insbesondere im pTX-Betrieb zu verwenden.

Die Gradientensteuerung 11, die HF-Sende-/Empfangseinheit 12 und die Empfangseinheit 13 für die Lokalspulen 6 werden jeweils koordiniert durch eine Messsteuereinheit 15 angesteuert. Diese sorgt durch entsprechende Befehle dafür, dass ein gewünschter Gradienten-Pulszug GP durch geeignete Gradientensteuersignale SGx, SGy, SGz ausgesendet wird, und steuert parallel die HF-Sende-/Empfangseinheit 12 so an, dass ein Mehrkanal-Pulszug MP ausgesendet wird, d. h. dass auf den einzelnen Sendekanälen S₁, ... S_{N} parallel die passenden Hochfrequenzpulse auf die einzelnen Sendestäbe der Ganzkörperspule 5 gegeben werden. Außerdem muss dafür gesorgt werden, dass zum passenden Zeitpunkt die Magnetresonanzsignale an den Lokalspulen 6 durch die HF-Empfangseinheit 13 bzw. eventuelle Signale an der Ganzkörperspule 5 durch die HF-Sende-/Empfangseinheit 12 ausgelesen und weiterverarbeitet werden. Die Messsteuereinheit 15 gibt die entsprechenden Signale, insbesondere den Mehrkanal-Pulszug MP an die Hochfrequenz-Sende-/Empfangseinheit 12 und den Gradienten-Pulszug GP an die Gradienten-Steuereinheit 11, gemäß einem vorgegebenen Steuerprotokoll P vor. In diesem Steuerprotokoll P sind alle Steuerdaten hinterlegt, die während einer Messung gemäß einer vorgegebenen Ansteuersequenz AS eingestellt werden müssen.

Üblicherweise sind in einem Speicher 16 eine Vielzahl von Steuerprotokollen P für verschiedene Messungen hinterlegt. Diese könnten über das Terminal 20 vom Bediener ausgewählt und gegebenenfalls variiert werden, um dann ein passendes Steuerprotokoll P für die aktuell gewünschte Messung zur Verfügung zu haben, mit dem die Messsteuereinheit 15 arbeiten kann. Im Übrigen kann der Bediener auch über ein Netzwerk NW Steuerprotokolle P, beispielsweise von einem Hersteller des Magnetresonanzsystems, abrufen und diese dann gegebenenfalls modifizieren und nutzen.

Der grundlegende Ablauf einer solchen Magnetresonanzmessung und die genannten Komponenten zur Ansteuerung sind dem Fachmann aber bekannt, so dass sie hier im Detail nicht weiter besprochen werden. Im Übrigen kann ein solcher Magnetresonanzscanner 2 sowie die zugehörige Steuereinrichtung 10 noch eine Vielzahl weiterer Komponenten aufweisen, die hier ebenfalls nicht im Detail erläutert werden. Es wird an dieser Stelle darauf hingewiesen, dass der Magnetresonanzscanner 2 auch anders aufgebaut sein kann, beispielsweise mit einem seitlich offenen Patientenraum, und dass im Prinzip die Hochfrequenz-Ganzkörperspule nicht als Birdcage-Antenne aufgebaut sein muss.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, der durch die Ansprüche definiert wird. So wurden das Verfahren und die Rekonstruktionseinrichtung in erster Linie anhand eines Magnetresonanzsystems zur Aufnahme von medizinischen Bilddaten erläutert. Die Erfindung ist jedoch nicht auf die Anwendung im medizinischen Bereich beschränkt, sondern die Erfindung kann auch grundsätzlich auf Magnetresonanzsysteme für die Aufnahme von dynamischen Bildern für andere Zwecke angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zum Rekonstruieren von dynamischen Bilddaten, aufweisend die Schritte:
- zeitabhängiges Akquirieren von Magnetresonanzsignalen als Rohdaten (RD) von einem Untersuchungsbereich (FoV) eines Patienten, wobei zumindest ein Teil der Rohdaten (RD) unterschiedlichen Werten von mindestens zwei Bewegungsparametern (BPₜ) zugeordnet sind, wobei es sich bei den mindestens zwei zeitabhängigen Bewegungsparametern um die Zeit und einen Atemzustand oder um die Zeit und einen Herzbewegungszustand handelt,
- Rekonstruieren von ersten zeitabhängigen Bilddaten (BD₁), auf Basis der akquirierten Rohdaten (RD),
- Ermitteln von Deformationsfeldern (DF) auf Basis der ersten Bilddaten (BD₁) in Abhängigkeit von den mindestens zwei zeitabhängigen Bewegungsparametern (BPₜ),
- Erzeugen von korrigierten Bilddaten (BDₖ) auf Basis der Deformationsfelder (DF), der Rohdaten (RD) und der ersten Bilddaten (BD₁),
wobei für das Erzeugen von korrigierten Bilddaten (BDₖ) auf Basis der Deformationsfelder (DF) für unterschiedliche Werte der mindestens zwei zeitabhängigen Bewegungsparameter (BPₜ) synthetisierte Rohdaten (RD_{S}) ermittelt werden und auf Basis der synthetisierten Rohdaten (RD_{S}) akquirierte Rohdaten (RD) geänderten Werten von Bewegungsparametern (BPₜ) neu zugewiesen werden.

2. Verfahren nach Anspruch 1, wobei das Erzeugen der korrigierten Bilddaten (BDₖ) auf Basis einer iterativen Rekonstruktion erfolgt, bei der die Deformationsfelder (DF) zur Erzeugung von korrigierten Rohdaten genutzt werden.

3. Verfahren nach Anspruch 1 oder 2, wobei das zeitabhängige Akquirieren von Rohdaten (RD) eine kartesische Abtastung von Rohdaten umfasst.

4. Verfahren nach einem der Ansprüche 1 bis 3, wobei das Ermitteln von Deformationsfeldern (DF) eine Registrierung von Bilddaten mit zumindest teilweise unterschiedlichen Werten der zeitabhängigen Bewegungsparameter (BPₜ) umfasst.

5. Verfahren nach einem der vorstehenden Ansprüche, wobei die Zuweisung zu geänderten Werten von Bewegungsparametern (BPₜ) in Abhängigkeit davon erfolgt, welche synthetisierten Rohdatenpunkte (RD_{S}) in einem Abstandsmaß oder mittels Korrelation am nächsten an den gemessenen Rohdatenpunkten (RD) liegen.

6. Verfahren nach Anspruch 5, wobei die Neuzuweisung der Rohdaten (RD) zunächst auf einer Untermenge der akquirierten Rohdaten (RD), vorzugweise in einer vollständig abgetasteten Richtung, erfolgt und anschließend eine Rekonstruktion der korrigierten Bilddaten (BDₖ) auf Basis der gesamten Rohdaten (RD) erfolgt.

7. Verfahren nach einem der vorstehenden Ansprüche, wobei Rohdaten (RD) welche sich in mindestens einem der mindesten zwei zeitabhängigen Bewegungsparameter (BPₜ) unterscheiden, verworfen werden und die korrigierten Bilddaten (BDₖ) nur in Abhängigkeit einer Untermenge der mindestens zwei zeitabhängigen Bewegungsparameter (BPₜ) erzeugt werden.

8. Bildrekonstruktionseinrichtung (30) aufweisend:
- eine Eingangsschnittstelle (31), die zum Empfangen von zeitabhängig akquirierten Magnetresonanzsignalen als Rohdaten (RD) von einem Untersuchungsbereich (FoV) eines Patienten ausgebildet ist,
wobei zumindest ein Teil der Rohdaten (RD) unterschiedlichen Werten von mindestens zwei Bewegungsparametern (BPₜ) zugeordnet sind, wobei es sich bei den mindestens zwei zeitabhängigen Bewegungsparametern um die Zeit und einen Atemzustand oder um die Zeit und einen Herzbewegungszustand handelt,
- eine Rekonstruktionseinheit (32), die zum Rekonstruieren von ersten zeitabhängigen Bilddaten (BD₁), auf Basis der akquirierten Rohdaten (RD) ausgebildet ist,
- eine Deformationsfeld-Ermittlungseinheit (33), die zum Ermitteln von Deformationsfeldern (DF) auf Basis der ersten Bilddaten (BD₁) in Abhängigkeit von den mindestens zwei zeitabhängigen Bewegungsparametern (BPₜ) ausgebildet ist,
- eine Korrektureinheit (34), die zum Erzeugen von korrigierten Bilddaten (BDₖ) auf Basis der Deformationsfelder (DF), der Rohdaten (RD) und der ersten Bilddaten (BD₁) ausgebildet ist,
wobei die Korrektureinheit (34) ausgebildet ist, für das Erzeugen von korrigierten Bilddaten (BDₖ) auf Basis der Deformationsfelder (DF) für unterschiedliche Werte der mindestens zwei zeitabhängigen Bewegungsparameter (BPₜ) synthetisierte Rohdaten (RD_{S}) zu ermitteln und auf Basis der synthetisierten Rohdaten (RD_{S}) akquirierte Rohdaten (RD) geänderten Werten von Bewegungsparametern (BPₜ) neu zuzuweisen.

9. Magnetresonanzbildgebungssystem (1), umfassend eine Bildrekonstruktionseinrichtung (30) nach Anspruch 8.

10. Computerprogrammprodukt mit einem Computerprogramm, welches direkt in eine Speichereinrichtung einer Steuereinrichtung (10) eines Magnetresonanzbildgebungssystems (1) ladbar ist, mit Programmabschnitten, um alle Schritte des Verfahrens nach einem der Ansprüche 1 bis 7 auszuführen, wenn das Computerprogramm in der Steuereinrichtung (10) des Magnetresonanzbildgebungssystems (1) ausgeführt wird.

11. Computerlesbares Medium, auf welchem von einer Rechnereinheit einlesbare und ausführbare Programmabschnitte gespeichert sind, um alle Schritte des Verfahrens nach einem der Ansprüche 1 bis 7 auszuführen, wenn die Programmabschnitte von der Rechnereinheit ausgeführt werden.

## Claims

1. Method for reconstructing dynamic image data, having the following steps:
- time-dependent acquisition of magnetic resonance signals as raw data (RD) from an examination region (FoV) of a patient,
wherein at least some of the raw data (RD) is assigned to different values of at least two movement parameters (BPt),
wherein the at least two time-dependent movement parameters are the time and a respiratory condition or the time and a heart movement condition,
- reconstruction of first time-dependent image data (BD₁), based on the acquired raw data (RD),
- ascertainment of deformation fields (DF) based on first image data (BD₁) as a function of the at least two time-dependent movement parameters (BPt),
- generation of corrected image data (BDₖ) based on deformation fields (DF), the raw data (RD) and the first image data (BD₁),
wherein for the generation of corrected image data (BDₖ) based on deformation fields (DF), raw data (RDₛ) synthesised for different values of the at least two time-dependent movement parameters (BPₜ) is ascertained and raw data (RD) acquired on the basis of synthesised raw data (RDₛ) is reallocated to changed values of movement parameters (BPt).

2. Method according to claim 1, wherein the generation of corrected image data (BDₖ) takes place on the basis of an iterative reconstruction in which the deformation fields (DF) are used to generate corrected raw data.

3. Method according to claim 1 or 2, wherein the time-dependent acquisition of raw data (RD) comprises a Cartesian scanning of raw data.

4. Method according to one of claims 1 to 3, wherein the determination of deformation fields (DF) comprises a registering of image data with at least partially different values of the time-dependent movement parameters (BPₜ).

5. Method according to one of the preceding claims, wherein the assignment to changed values of movement parameters (BPₜ) takes place as a function of which synthesised raw data points (RDₛ) are closest to the measured raw data points (RD) in a distance measurement or by means of correlation.

6. Method according to claim 5, wherein the reassignment of the raw data (RD) is first performed on a subset of the acquired raw data (RD), preferably in a fully sampled direction, and then the corrected image data (BDₖ) is reconstructed on the basis of all the raw data (RD).

7. Method according to one of the preceding claims, wherein raw data (RD) which differs in at least one of the at least two time-dependent movement parameters (BPₜ) is discarded and the corrected image data (BDₖ) is only generated as a function of a subset of the at least two time-dependent movement parameters (BPₜ).

8. Image reconstruction apparatus (30) having:
- an input interface (31), which is designed for receiving magnetic resonance signals acquired in a time-dependent manner as raw data (RD) from an examination region (FoV) of a patient, wherein at least some of the raw data (RD) is associated with different values of at least two movement parameters (BPt), wherein the at least two time-dependent movement parameters are the time and a respiratory condition or the time and a heart movement condition,
- a reconstruction unit (32), which is designed for reconstructing first time-dependent image data (BD₁) based on acquired raw data (RD),
- a deformation field determination unit (33), which is designed for determining deformation fields (DF) based on first image data (BD₁) as a function of at least two time-dependent movement parameters (BPt),
- a correction unit (34), which is designed for generating corrected image data (BDₖ) based on deformation fields (DF), raw data (RD) and first image data (BD₁),
wherein the correction unit (34) is designed, for the generation of corrected image data (BDₖ) based on deformation fields (DF), to ascertain raw data (RD_{S}) synthesised for different values of the at least two time-dependent movement parameters (BPₜ) and to reallocate raw data (RD) acquired on the basis of synthesised raw data (RDₛ) to changed values of movement parameters (BPt).

9. Magnetic resonance imaging system (1), comprising an image reconstruction apparatus (30) according to claim 8.

10. Computer program product with a computer program which can be loaded directly into a storage device of a control device (10) of a magnetic resonance imaging system (1), with program sections to perform all the steps of the method according to one of claims 1 to 7 when the computer program is executed in the control device (10) of the magnetic resonance imaging system (1).

11. Computer-readable medium on which program sections readable and executable by a processing unit are stored to perform all the steps of the method according to one of claims 1 to 7 when the program sections are executed by the processing unit.

## Revendications

1. Procédé de reconstruction de données d'image dynamiques comportant les stades :
- acquisition en fonction du temps de signaux de résonnance magnétique sous la forme de données (RD) brutes d'une partie (FoV) à examiner d'un patient, dans lequel au moins une partie des données (RD) brutes sont associées à des valeurs différentes d'au moins deux paramètres (BPₜ) de mouvement, dans lequel les au moins deux paramètres de mouvement en fonction du temps sont le temps et un état respiratoire ou le temps et un état de mouvement du cœur,
- reconstruction de premières données (BD₁) d'image en fonction du temps, sur la base des données (RD) brutes acquises,
- détermination de champs (DF) de déformation sur la base des premières données (BD₁) d'image en fonction des au moins deux paramètres (BPₜ) de mouvement en fonction du temps,
- production de données (BDₖ) d'image corrigées sur la base des champs (DF) de déformation, des données (RD) brutes et des premières données (BD₁) d'image,
dans lequel, pour la production de données (BDₖ) d'image corrigées, on détermine des données (RDₛ) brutes synthétisées sur la base des champs (DF) de déformation pour des valeurs différentes des au moins deux paramètres (BPₜ) de mouvement en fonction du temps, et des données (RD) brutes, acquises sur la base des données (RDₛ) brutes synthétisées, sont affectées à nouveau à des valeurs modifiées de paramètres (BPₜ) de mouvement.

2. Procédé suivant la revendication 1, dans lequel la production des données (BDₖ) d'image corrigées s'effectue sur la base d'une reconstruction par itération, dans laquelle on utilise les champs (DF) de déformation pour la production des données brutes corrigées.

3. Procédé suivant la revendication 1 ou 2, dans lequel l'acquisition en fonction du temps de données (RD) brutes comprend un échantillonnage cartésien de données brutes.

4. Procédé suivant l'une des revendications 1 à 3, dans lequel la détermination de champs (DF) de déformation comprend un enregistrement de données d'image ayant des valeurs différentes au moins en partie des paramètres (BPₜ) de mouvement en fonction du temps.

5. Procédé suivant l'une des revendications précédentes, dans lequel l'affectation à des valeurs modifiées de paramètres (BPₜ) de mouvement s'effectue en fonction des points (RDₛ) de données brutes synthétisés, qui sont, à une certaine distance ou au moyen d'une corrélation, les plus proches des points (RD) de données brutes mesurés.

6. Procédé suivant la revendication 5, dans lequel l'affectation à nouveau des données (RD) brutes s'effectue d'abord sur un sous-ensemble des données (RD) brutes acquises, de préférence dans une direction échantillonnée complètement, et il s'effectue ensuite une reconstruction des données (BDₖ) d'image corrigées sur la base de toutes les données (RD) brutes.

7. Procédé suivant l'une des revendications précédentes, dans lequel on rejette des données (RD) brutes, qui se distinguent par au moins l'un des au moins deux paramètres (BPₜ) de mouvement en fonction du temps et on ne produit les données (BDₖ) d'image corrigées qu'en fonction d'un sous-ensemble des au moins deux paramètres (BPₜ) de mouvement en fonction du temps.

8. Dispositif (30) de reconstruction d'image comprenant :
- une interface (31) d'entrée, qui est constituée pour la réception de signaux de résonnance magnétique acquis en fonction du temps, comme données (RD) brutes d'une partie (FoV) en examen d'un patient,
dans lequel au moins une partie des données (RD) brutes sont affectées à des valeurs différentes d'au moins deux paramètres (BPₜ) de mouvement, dans lequel les au moins deux paramètres de mouvement en fonction le temps sont le temps et un état respiratoire ou le temps et un état de mouvement cardiaque,
- une unité (32) de reconstruction, qui est constituée pour la reconstruction de premières données (BD₁) d'image en fonction du temps, sur la base des données (RD) brutes acquises,
- une unité (33) de détermination de champs de déformation, qui est constituée pour la détermination de champs (DF) de déformation sur la base des premières données (BD₁) d'image en fonction des au moins deux paramètres (BPₜ) de mouvement en fonction du temps,
- une unité (34) de correction, qui est constituée pour la production de données (BDₖ) d'image corrigées sur la base des champs (DF) de déformation, des données (RD) brutes et des premières données (BD₁) d'image,
dans lequel l'unité (34) de correction est constituée pour déterminer, pour la production de données (BDₖ) d'image corrigées, des données (RDₛ) brutes synthétisées sur la base des champs (DF) de déformation pour des valeurs différentes des au moins deux paramètres (BPₜ) de mouvement en fonction du temps, et pour affecter à nouveau des données (RD) brutes, acquises sur la base des données (RDₛ) brutes synthétisées à des valeurs modifiées de paramètres (BPₜ) de déplacement.

9. Système (1) d'imagerie par résonnance magnétique, comprenant un dispositif (30) de reconstruction d'image suivant la revendication 8.

10. Produit de programme d'ordinateur comprenant un programme, qui peut être chargé directement dans un dispositif de mémoire d'un dispositif (10) de commande d'un système (1) d'imagerie par résonnance magnétique, comprenant des parties de programme, pour exécuter tous les stades du procédé suivant l'une des revendications 1 à 7, lorsque le programme d'ordinateur est exécuté dans le dispositif (10) de commande du système (1) d'imagerie par résonnance magnétique.

11. Support, déchiffrable par ordinateur, sur lequel sont mises en mémoire des parties de programme déchiffrables et exécutables par une unité informatique pour exécuter tous les stades du procédé suivant l'une des revendications 1 à 7, lorsque les parties de programme sont exécutées par l'unité informatique.
